Europäisches Patentamt

European Patent Office

Office européen des brevets

(19)

(11) Publication number: **0 228 357 B1**

## EUROPEAN PATENT SPECIFICATION

(12)

(45) Date of publication of patent specification: **17.06.92**    (51) Int. Cl.⁵: **C07K 15/14**, C07K 3/02, C12N 5/00

(21) Application number: **86870196.2**

(22) Date of filing: **29.12.86**

The file contains technical information submitted after the application was filed and not included in this specification

(54) **Prostate-derived growth factor.**

(30) Priority: **02.01.86 US 815685**

(43) Date of publication of application:
**08.07.87 Bulletin 87/28**

(45) Publication of the grant of the patent:
**17.06.92 Bulletin 92/25**

(84) Designated Contracting States:
**AT BE CH DE FR GB IT LI LU NL SE**

(56) References cited:
**EP-A- 0 128 849**
**US-A- 4 054 557**

**PROCEEDINGS OF THE NATIONAL ACADEMY OF SCIENCES OF THE USA, vol. 83, no. 21, November 1986, pages 8162-8166, Washington, DC, US; S. MAEHAMA et al.: "Purification and partial characterization of prostate-derived growth factor"**

**BIOCHEMICAL JOURNAL, vol. 218, no. 2, 1984, pages 563-571, GB; A. HIIPAKKA et al.: "Immunochemical characterization of the androgen-dependent spermine-binding protein of the rat ventral prostate"**

(73) Proprietor: **WASHINGTON UNIVERSITY**
**Lindell and Skinker Boulevards**
**St. Louis, Missouri 63130(US)**

(72) Inventor: **Deuel, Thomas Franklin**
**81 Aberdeen Place**
**Clayton Missouri 63105(US)**

(74) Representative: **McLean, Peter et al**
**MONSANTO SERVICES INTERNATIONAL SA/NV Patent Department Avenue de Tervuren 270-272 Letter Box No. 21**
**B-1150 Brussels(BE)**

DISSERTATION ABSTRACTS INTERNATION-AL, vol. 44, no. 7, January 1984, page 2044, US; L.L. CHAMBERLIN et al.: "Isolation and characterization of a 20K protein from rat ventral prostate"

THE JOURNAL OF UROLOGY, vol. 132, no. 6, 1984, pages 1212-1215, The Williams & Wilkins Co., US; M.T. STORY et al.: "Partial purification of a prostatic growth factor"

PROGRESS IN CLINICAL AND BIOLOGICAL RESEARCH, vol. 145, 1984, pages 197-216, Alan R. Liss, Inc., New York, US; M.T. STOREY et al.: "Preliminary characterization and evaluation of techniques for the isolation of prostate-derived growth factor"

THE JOURNAL OF UROLOGY, vol. 131, no. 4, part 2, no. 81, 1984, page 124A, US; D.W. WARREN et al.: "Fibroblast mitogenic activity of the Copenhagen rat prostate and prostate derived tumors"

BIOCHEMICAL AND BIOPYHSICAL RESEARCH COMMUNICATIONS, vol. 132, no. 3, 15th November 1985, pages 1103-1109, Academic Press, Inc., New York, US; N. NISHI et al.: "A human prostatic growth factor (hPGF): partial purification and characterization"

DISSERTATION ABSTRACTS INTERNATION-AL B, vol. 44, no. 5, November 1983, page 1317, US; J.S. RUBIN: "Purification and characterization of three polypeptide growth factors from mammalian sources"

PROGRESS IN CLINICAL AND BIOLOGICAL RESEARCH, vol. 145, 1984, pages 181-195, Alan R. Liss, Inc., New York, US; R.F. PARRISH et al.: "Prostate-derived growth factors"

**Description**

Background of the Invention

This invention relates to a novel glycoprotein growth factor and, more particularly, to a prostate-derived growth factor (PrGF).

In recent years a considerable number of growth factors derived from various animal cells have been isolated and characterized. Illustrative of these growth factors are nerve growth factor (NGF) which has been purified from several different cell sources, insulin-like growth factors (IGF-I and IGF-II), epidermal growth factor (EGF), fibroblast growth factor (FGF), platelet-derived growth factor (PDGF), endothelial cell growth factor (ECGF), somatomedins and transforming growth factors (TGF) derived from various tumors and virally transformed cells. For background information on these growth factors see, for example, the recent brief review article by Kris et al., Biotechnology, February 1985, pp. 135-140; and the comprehensive review in Hormonal Proteins and Peptides, Ed. by Choh Hao Li, Vol. 12, "Growth Factors," Academic Press, 1984.

These growth factors, many of which are Polypeptide hormone-like agents in structure, function to regulate the proliferation, differentiation and survival of animal cells. The isolation and characterization of these growth factors is of major importance for understanding the regulation of normal tissue specific DNA synthesis and cell division, as well as for understanding abnormal mechanisms of cell growth such as observed in atherosclerosis and in neoplasia. Growth factor activity in prostate glands may be especially important to characterize because benign prostatic hypertrophy and prostate cancer are common tumors in males and because prostatic cancer metastatic to bone is nearly unique in inducing osteoblasts to form new bone locally. Thus, trophic factors that are secreted by prostatic tissue may be important not only in the normal growth and differentiation of prostatic tissue but also as mediators of abnormal prostatic growth.

The concept of a trophic factor released by metastatic prostatic carcinoma cells in bone has been suggested heretofore. See Franks, J. Path. Bact. 72, 603-611 (1956); Cook et al, J.Urol. 99, 87-96 (1968); Galasko, J. Bone Joint Surg. 57B, 353-359 (1973); Warren et al, Arch. Pathol. 22, 139-160 (1936); and Rosai, in Pathology, eds. Anderson and Kissane, C. V. Mosby Co., St. Louis, 7th. ed., 1977, pp. 1978-2014. Growth factor activity in extracts of prostatic tissue have been previously demonstrated but not purified or characterized. See Lawson et al, in The Prostatic Cell: Structure and Function, Part A, eds. Murphy et al, Alan R. Liss, Inc., N.Y. 1981, pp. 325-336; Story et al, J. Urol. 130, 175-179 (1983); and Jacobs et al, Urol. 16, 488-491 (1980). A mRNA fraction in human prostatic cancer cells coding for a novel osteoblast-stimulating factor having a molecular weight of ~ 20,000 has recently been identified and associated with this activity by Simpson et al, Endocrinology 117, 1615-1620 (1985).

Brief Description of the Invention

In accordance with the present invention a novel glycoprotein growth factor has been isolated from prostate gland of rats and characterized as follows:

The prostate-derived growth factor (PrGF) is an acidic glycoprotein with an apparent molecular weight of about 25 kDa as determined by nonreduced sodium dodecylsulfate polyacrylamide gel electrophoresis (SDS-PAGE). It is acid- and heat-stable and destroyed by protease activity with trypsin and pronase. Its isolectric point is about 5.0 (pI ~pH 5.0) as determined by isolectric focusing. PrGF is further characterized by its sensitivity to reduction with $\beta$-mercaptoethanol and by the absence of transforming growth factor activity. Thus, reduction by $\beta$-mercaptoethanol results in the disappearance of the band at 25 kDa, loss of >90% mitogenic activity, and the appearance of a new protein band of 6-8 kDA. PrGF thus appears to be a 25 kDa protein with subunits of 6-8 kDa stabilized by disulfide bonds. The glycoprotein nature of PrGF is indicated by its weak staining with silver in SDS-PAGE gels, by the labeling of PrGF with tritium by use of sodium metaperiodate -Na[$^3$H]BH$_4$ and by the appearance of an unidentified peak on amino acid analysis consistent with an amino sugar.

The purified PrGF has been demonstrated to have mitogenic activity toward normal rat kidney fibroblast cells (NRK). It induces the linear incorporation of [methyl-$^3$H]-thymidine into NRK cells over a concentration range of about 0-16 ng/ml. PrGF also stimulates the incorporation of [methyl-$^3$H]-thymidine into DNA of isolated osteoblasts from fetal rat calvaria, which indicates that PrGF may mediate the osteoblastic proliferation characteristic of metastatic prostatic cancer to bone.

The PrGF of this invention thus appears to be unique and is a potential and potent mediator of prostatic activities related to abnormal prostatic growth.

Detailed Description of the Invention

While the specification concludes with claims particularly pointing out and distinctly claiming the subject matter regarded as forming the present invention, it is believed that the invention will be better understood from the following detailed description of preferred embodiments of the invention taken in connection with the accompanying drawings in which briefly:

FIG. 1 is a graphical representation of the elution profile of acid extracted rat prostate tissue after its partial purification by a series of ion exchange and gel permeation chromatographic separations.

FIG. 2 is a graphical representation of the elution profile of the peak II (76-81 ml) material from FIG. 1 subjected to reverse phase high performance liquid chromatography (HPLC).

FIG. 3 shows the SDS-PAGE pattern of the purified prostate growth factor (PrGF) isolated in FIG. 2 (11-13 ml peak).

FIG. 4 is a graphical representation of the mitogenic activity of the PrGF eluted from the sliced gels of FIG. 3.

FIG. 5 is a graphical representation of the isoelectric focusing of the PrGF from FIG. 4 showing the pI ~ 5.0.

FIG. 6 is a graphical representation of the mitogenic dose response of the purified PrGF from FIG. 4 on NRK cells.

FIG. 7 is a graphical representation of the mitogenic time course of the purified PrGF from FIG. 4 on NRK cells.

Hereinafter the symbol "*" means that the related term is a trade mark or a registered trade mark.

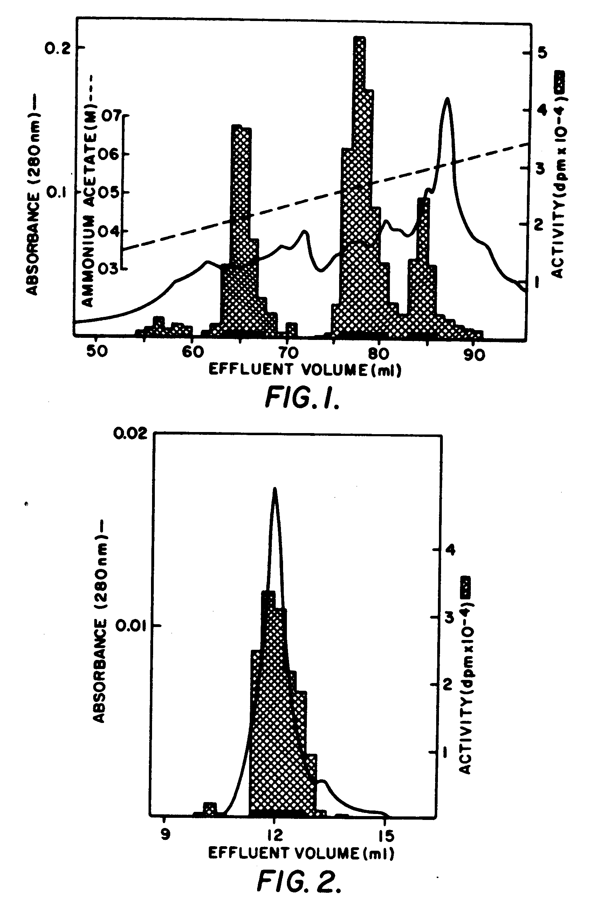

The source material used for purifying the PrGF illustrated by the results shown in FIGS. 1 to 7 was frozen rat prostates obtained commercially from Pel-Freez Biologicals (Rogers, Arkansas). The initial material was homogenized and acid extracted with acetic acid and then purified 16,400 fold over the initial extracts by a series of purification steps. Thus, the acid extracted material was first subjected to ion exchange chromatography using SP-Sephadex® C-25* (sulphopropyl functional groups) and DEAE-Sephadex A-50* (diethylaminoethyl functional groups) columns. The active fractions were then concentrated by 70% ammonium sulfate precipitation followed by gel permeation chromatography with Sephadex G-75*. The Sephadex* (cross-linked dextran) based materials used above are well-known chromatographic materials commercially available from Pharmacia Fine Chemicals AB (Uppsala, Sweden).

The active fractions from the gel permeation chromatography were subjected to ion exchange HPLC using a TSK-DEAE-5-PW* column commercially available from Bio-Rad (Richmond, California) followed by reverse phase HPLC on a Vydac $C_{18}$* silica column commercially available from The Separations Group (Hesperia, California). The active material from the reverse phase HPLC was then subjected to another HPLC step using a TSK-G2000SW* gel permeation column commercially available from LKB (Bromma, Sweden) to produce the final 16,400 fold purified PrGF.

In order to illustrate specific preferred embodiments of the invention in greater detail, the following exemplary laboratory preparative work was carried out.

## EXAMPLE

### Protein Purification

Nine hundred gm of rat prostate were homogenized at 4°C in 1500 ml of 1.0 M acetic acid, centrifuged at 13,500 x g for 30 min. and the precipitate re-extracted in 1500 ml of 1.0 M acetic acid. The pooled extracts were dialyzed against 10 mM ammonium acetate (pH 5.5). All subsequent purification steps were performed at 4°C except for HPLC.

The acid extracts were loaded on a 4.5 cm x 45 cm SP-Sephadex C-25* column (bed volume 700 ml) equilibrated with 10 mM ammonium acetate (pH 5.5) and eluted with 10 mM ammonium acetate (pH 5.5). The active fractions were pooled and adjusted to pH 7.0 with ammonium hydroxide and loaded on a 4.5 cm x 30 cm DEAE Sephadex A-50* column (bed volume 480 ml) equilibrated with 10 mM ammonium acetate (pH 7.0). The PrGF activity did not bind under these conditions and was eluted with the flow through fractions. The active fractions were pooled and concentrated with 70% ammonium sulfate and the precipitate was dialyzed against 1.0 M acetic acid and lyophilized.

The lyophilized sample was dissolved in 50 ml of 1.0 M acetic acid and loaded on a 5 cm x 90 cm Sephadex G-75* column (fine, 200-400 mesh, bed volume 1550 ml) equilibrated with 1.0 M acetic acid. The column was eluted with 1.0 M acetic acid and the active fractions were pooled (250 ml), lyophilized, and 6.3 mg of the lyophilized sample (~ 33% of total protein) was dissolved in 5 ml of 10mM ammonium acetate (pH 7.0) and loaded onto a Bio Gel TSK-DEAE-5-PW* column (21.5 mm x 150 mm) equilibrated with 10 mM ammonium acetate (pH 7.0). The column was eluted with a linear ammonium acetate gradient (0.01-0.8 M) and fractions were assayed for biological activity.

The fractions corresponding to each of three activity peaks were collected and labeled I, II, and III in order of elution, dialyzed against 1.0 M acetic acid, and lyophilized.

Peak II samples from separate DEAE-HPLC runs were dissolved in 1.5 ml of 0.1% trifluoroacetic acid and loaded onto a 4.6 mm x 150 mm $C_{18}$* column (particle size, 5 $\mu$m; pore size, 300 A) equilibrated with 0.1% trifluoroacetic acid, and eluted with a linear gradient of 0-80% acetonitrile in 0.1% trifluoroacetic acid. The active fractions were pooled, dried by Speed Vac* (Savant Instruments), dissolved in 100 $\mu$l of 50 mM sodium acetate (pH 5.5), 0.2 M NaCl, and injected into a 7.5 mm x 300 mm TSK G2000 SW* column (particle size, 10 $\mu$m) equilibrated with the same buffer. The column was eluted under isocratic conditions, fractions assayed for mitogenic activity, the active fractions pooled and dialyzed against 1.0 M acetic acid, and stored at -20°C.

## Mitogenic Assays

The mitogenic activity of PrGF was assayed by incorporation of [methyl-$^3$H]-thymidine into acid-insoluble DNA of normal rat kidney (NRK) cells obtained from the American Type Culture Collection, Rockville, Maryland. NRK cells were maintained in Dulbecco's modified Eagle's medium* (DMEM) (K.C. Biologicals) containing 10% fetal calf serum, 100 $\mu$g/ml penicillin, and 100 $\mu$g/ml streptomycin and split into 48-well cell culture cluster plates (Costar) for assay. Samples (1-20 $\mu$l) to be tested for mitogenic activity were diluted to 0.3 ml with phosphate buffered saline (PBS) (pH 7.3) and mixed with 1.5 ml of medium consisting of DMEM, 6% plasma derived serum (PDS), and [methyl-$^3$H]-thymidine (specific activity 62.5 mCi/mmol) and 0.75 ml of the sample media was used. The PDS was prepared from human platelet poor Plasma by the method of Vogel et al., Proc. Natl. Acad. Sci. USA 75, 2810-2814 (1978). After incubating the cells for 24 hrs, the sample medium was aspirated and the cells were washed twice with 0.8 ml of PBS and incubated with 0.8 ml of 10% trichloroacetic acid (TCA) for 15 min. The acid-insoluble material was washed with 0.8 ml of ether-ethanol (1:2) and dissolved in 0.3 ml of 0.4 N sodium hydroxide. 0.2 ml aliquots were mixed with 5 ml of Dimilume 30* (Packard Instrument Co., Inc., Downers Grove, Illinois) and counted in a liquid scintillation spectrometer. The activity from 0.3 ml PBS was used as background and subtracted from all other values. Assays were done in duplicate.

## SDS-polyacrylamide Gel Electrophoresis

Fifteen percent SDS-PAGE was carried out according to the method of Laemmli, Nature 227, 680-685 (1970). Gels were stained with silver after fixation with 50% ethanol for 30 min in 100 ml of a solution of 0.8% silver nitrate, 0.02 M sodium hydroxide, and 0.22 M ammonium hydroxide. The gels were developed in 0.27 mM citric acid, 0.02% formaldehyde for 5-10 min and the reaction was stopped with 100 ml of 10% acetic acid.

To determine mitogenic activity in gels, 2 mm gel slices were incubated in l ml of 1.0 M acetic acid for 8-10 hrs, transferred into 2 ml of 70% formic acid and incubated for another 8-10 hrs. The 70% formic acid extracts were dried and assayed for mitogenic activity.

## Isoelectric Focusing

Isoelectric focusing was carried out in 7.5% polyacrylamide gels containing 2% ampholyte (ampholine*, pH 3.5-10; LKB) with 0.02 M phosphoric acid at the anode and 1.0 M sodium hydroxide at the cathode. The lyophilized samples were dissolved in 30% glycerol and 2% ampholyte and were run at a constant current of 50 mA until 250 V was reached and continued at 250 V until the dye marker (methyl red, pI 3.75) no longer migrated. The pH gradient was assayed after soaking 2 mm gel slices in 2 ml of HPLC-grade water for 8-10 hrs. Mitogenic activity was assayed as described above after soaking gel slices in 1.5 ml 1.0 M acetic acid for 8-10 hrs, dialysis of each extract against 1.0 M acetic acid, and concentration of each extract by Speed Vac*.

## Amino Acid Analysis

Amino acid analyses were performed as adapted from Ishida et al J. Chromatogr. 204, 143-148 (1981), using the Waters Amino Acid Systems* (Waters Associates, Milford, MA), and using 80 pMoles PrGF.

## Stability Studies

Stability to boiling was tested after incubating 5 ng of purified PrGF dissolved with 0.1 ml PBS in boiling water for 5 min and rapid chilling on ice.

Reduced PrGF was tested after 10 ng of purified PrGF (in 0.5 ml PBS) was incubated in 0.1 M $\beta$-mercaptoethanol at 100°c for 3 min, quick chilled on ice, and dialyzed against PBS. The control sample was treated in an identical manner (but without $\beta$-mercaptoethanol) and the percent activity of the treated sample to the untreated sample measured.

The effects of protease digestion were tested with 5 ng of purified PrGF incubated with either trypsin (50 or 100 $\mu$g/ml) or pronase (100 or 500 $\mu$g/ml) in 90 $\mu$l of PBS for 3 hrs at 37°C. The reactions were terminated with 10 $\mu$l of 1.0 M acetic acid and 3 min incubation in boiling water. Inactivated proteases were studied after incubating 100 $\mu$g/ml of trypsin or 500 $\mu$g/ml of pronase in 90 $\mu$l of PBS and 10 $\mu$l of 1.0 M acetic acid at 100°C for 3 min.

### Iodination of PrGF

Purified PrGF (2.7 $\mu$g) was iodinated by glucose oxidase-lactoperoxidase in the presence of 2 mCi carrier-free Na[$^{125}$I], using a radioiodination kit (New England Nuclear). Iodinated PrGF was extensively dialyzed against 10 mM sodium phosphate (pH 7.2) and stored at -20°C.

### Protein Determination

Protein concentrations were determined by the method of Lowry, J. Biol. Chem. 193, 265-275 (1951), using bovine serum albumin as standard. The concentration of purified PrGF was determined by amino acid analysis.

The results of the above laboratory preparative work leading to the purification of the novel PrGF of this invention and demonstration of its biological activity are further exemplified by the following detailed description of FIGS. 1 to 7 of the drawings and Tables 1 to 4, set forth below.

### FIG. 1

Preparative DEAE HPLC. A 6.3 mg lyophilized sample from Sephadex G-75* was dissolved in 5 ml of 10 mM ammonium acetate (pH 7.0). The sample was loaded on a 21.5 mm x 150 mm TSK-DEAE 5-PW* column (particle size, 10 $\mu$M; pore size 1000 A) equilibrated with 10 mM ammonium acetate (pH 7.0). The column was eluted with a 0.01-0.8 M linear gradient of ammonium acetate (pH 7.0) for 4 hours at 22°C. The flow rate was 0.5 ml/min and 1 ml samples were collected. Mitogenic activity was assayed using 2 $\mu$l from each fraction. The active fractions from effluent volumes 64-68 ml, 76-81 ml, and 84-86 ml were pooled separately and labeled peak I, peak II, and peak III, respectively. Each peak was dialyzed against 1 M acetic acid and lyophilized.

### FIG. 2

TSK G2000 SW* HPLC. The active pool from the reverse phase $C_{18}$* HPLC was lyophilized and dissolved in 100 $\mu$1 of 50 mM sodium acetate (pH 5.5), 0.2 M NaCl buffer. The sample was loaded on a 7.5 mm x 300 mm TSK G2000 SW* column (particle size, 10 $\mu$m) equilibrated with 50 mM sodium acetate (pH 5.5), 0.2 M NaCl buffer. The column was eluted under isocratic conditions using 50 mM sodium acetate (pH 5.5), 0.2 M NaCl buffer. The flow rate was 0.3 ml/min and 0.3 ml samples were collected. Mitogenic activity was assayed using 1 $\mu$l from each fraction. The fractions corresponding to elution volumes 11-13 ml were pooled, dialyzed against 1.0 M acetic acid, and stored at -20°C.

### FIG. 3

SDS-polyacrylamide gel electrophoresis of purified PrGF. Purified PrGF was analyzed by SDS-PAGE (15% acrylamide separating gel, 4% acrylamide stacking gel). The gel was run at a constant voltage and stained as described above. Lane 1: non-reduced PrGF (540 ng); Lane 2: non-reduced PrGF (190 ng); Lane 3: reduced PrGF (540 ng); Lane 4: reduced PrGF (190 ng). The molecular weight standards used were phosphorylase B (92.5 kDa), bovine serum albumin (66.2 kDa), ovalbumin (45 kDa), carbonic anhydrase (31 kDa), soybean trypsin inhibitor (21.5 kDa) and lysozyme (14.4 kDa). Reduction was by 2-mercaptoethanol (2-ME).

FIG. 4

The biological activity of PrGF eluted from SDS-PAGE. Twenty ng of purifed PrGF was applied to 15% SDS-PAGE. The sample was pretreated in the presence of (0—0) or in the absence of (●—●) 5% $\beta$-mercaptoethanol. After electrophoresis, the gel was sliced into 2 mm sections and the biological activity from the sliced gels was eluted and measured as described above. Each arrow indicates a molecular weight standard in kDa.

FIG. 5

Isoelectric focusing in a 7.5% polyacrylamide gel. One hundred eight ng of purified PrGF with $^{125}$I-PrGF (5,000 cpm) was tested by isoelectric focusing as described above. Mitogenic activity from sliced gels was measured by [methyl-$^3$H]-thymidine incorporation (□). Migration of iodinated PrGF was determined by direct counting of sliced gels (●—●). The pH gradient was measured after soaking sliced gels in HPLC-grade water for 8 hours (0---0).

FIG. 6

Mitogenic dose-response of purified PrGF. Incorporation of [methyl-$^3$H]-thymidine into DNA of NRK cells was measured with increasing concentrations of purified PrGF in 6% PDS-DMEM. The average background value without added PrGF (11,947 dpm) was subtracted from the values with PrGF. Full stimulation by 17% human serum was 76,724 dpm.

FIG. 7

Mitogenic time course of purified PrGF on NRK cells. Confluent NRK cells were stimulated either with 5 ng/ml purified PrGF or with 17% human serum in 6% PDS-DMEM. A control assay of NRK cells used 6% PDS-DMEM alone. Incorporated [methyl-$^3$H]-thymidine into acid-precipitable DNA was measured and the control values subtracted from PrGF stimulated cells (●—●) or human serum stimulated cells (0----0).

The purification of the novel PrGF and demonstration of its mitogenic activity is thus evidenced from the above by explanation as follows:

Protein Purification

One molar acetic acid extracts of rat prostate were dialyzed against 10 mM ammonium acetate (pH 5.5). The mitogenic activity did not bind to a SP-Sephadex* column but 64% of the mitogenic activity was recovered in the flow through fractions with a 12.5-fold purification. The fractions from SP-Sephadex* column chromatography containing the activity were pooled and equilibrated to pH 7.0 and passed through a DEAE-Sephadex* column with a recovery of 104% and an 87-fold purification.

The active fractions from DEAE-Sephadex* were pooled, concentrated by ammonium sulfate precipitation (70% recovery, 97-fold purification) and the precipitate was resuspended in 1 M acetic acid (255 mg protein); the solubilized precipitate was fractionated on a Sephadex G-75* column and the active fractions collected. After lyophilization, the active fractions were loaded onto a DEAE-HPLC column, equilibrated with 10 mM ammonium acetate (pH 7.0), and eluted with a linear gradient (0.01-0.8 M ammonium acetate, pH 7.0). The elution profile of the DEAE-HPLC column (FIG. 1) demonstrated three peaks of mitogenic activity. Peak II contained the largest amount of mitogenic activity and eluted at ~ 0.50 M ammonium acetate. Peak I eluted at ~ 0.43 M ammonium acetate and peak III at ~ 0.56 M, with recoveries of mitogenic activity of ~ 56%, ~ 22%, and ~ 22%, respectively. The active peak II pool alone was used in subsequent purification.

The protein was then applied to a reverse phase $C_{18}$* HPLC column and a single activity peak eluted at ~ 45% acetonitrile, 0.1% trifluoroacetic acid, in a linear 0-80% acetonitrile gradient. The final purification was achieved using a TSK G2000* HPLC gel permeation column. As shown in FIG. 2, a sharp single protein peak was found with near constant specific growth factor activity, resulting in ~ 16,400-fold purification. The results are summarized in Table 1, below.

Sodium Dodecyl Sulfate Polyacrylamide Gel Electrophoresis (SDS-PAGE)

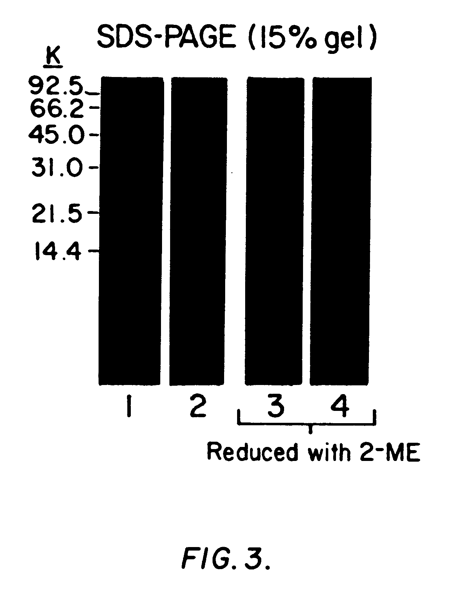

The purity of PrGF from TSK G2000* HPLC was analyzed by SDS-PAGE in 15% acrylamide and identification of protein bands by silver staining (FIG. 3). The intensity of the single silver stained bands was found to be approximately proportional to the amounts of protein. The protein was weakly stained and migrated with an apparent molecular mass of 25 kDa; no other bands were observed except for an artifactual band between the 66 kDa and 45 kDa markers.

After reduction with 5% $\beta$-mercaptoethanol, the protein band at 25 kDa disappeared and a broad darkly stained protein band was found at 6-8 kDa (lane 3 and lane 4). The same amounts of protein in lanes 1 and 2 were applied in lanes 3 and 4, respectively. Artifactual bands were also seen near the top of the gel.

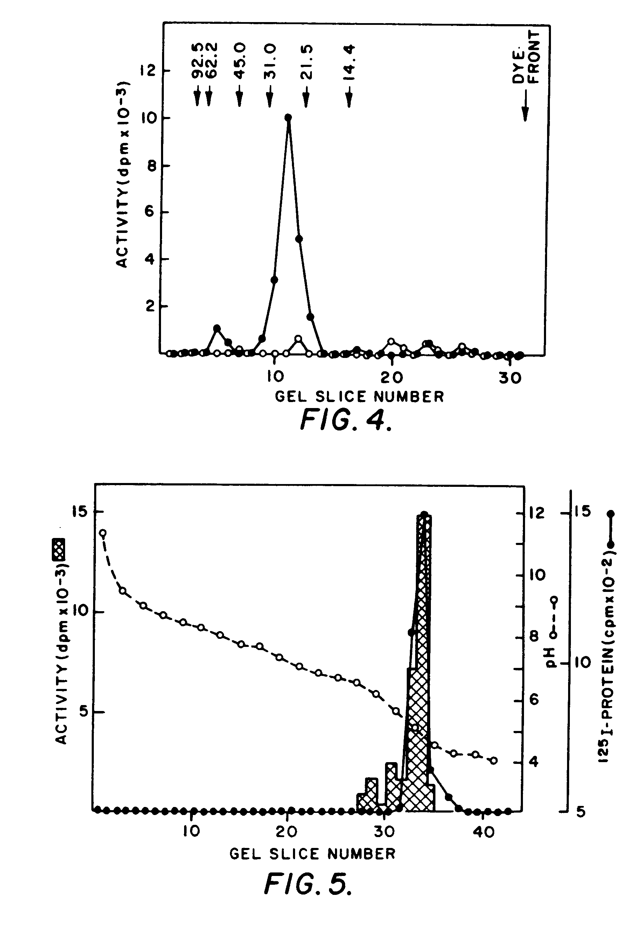

The mitogenic activity in the sliced gels was measured as described above. incubation of gels with 70% formic acid recovered more than 50% of the applied activity as measured after lyophilization. The results of assays of mitogenic activity from both non-reduced and reduced gels are shown in FIG. 4. A single activity peak was observed from non-reduced gels which corresponded precisely to the protein staining band at 25 kDa; no activity was recovered from reduced gels.

## Isoelectric Focusing of PrGF

Polyacrylamide gels (7.5%, 2% ampholyte, LKB) were used for isoelectric focusing. Because of limited amounts of protein available and the limited sensitivity of PrGF to silver staining, $^{125}$I-PrGF was used to localize PrGF in the isoelectric focusing gel and to compare the distribution of $^{125}$I-PrGF with the biological activity extracted from the isoelectric focusing gel.

As shown in FIG. 5, $^{125}$I-PrGF was identified at ~ pH 5.0. In precise agreement with the migration of $^{125}$I-PrGF, the peak of mitogenic activity was also found at ~ pH 5.0.

## Amino Acid Analysis of PrGF

The amino acid composition of PrGF was analyzed and is set forth in Table 2, below. The amino acid composition is consistent with the pI determined. A peak was noted in the chromatographic analysis consistent with an amino sugar, suggesting that PrGF is a glycoprotein. This peak has not been further characterized.

## Requirements of PrGF for Mitogenic Activity

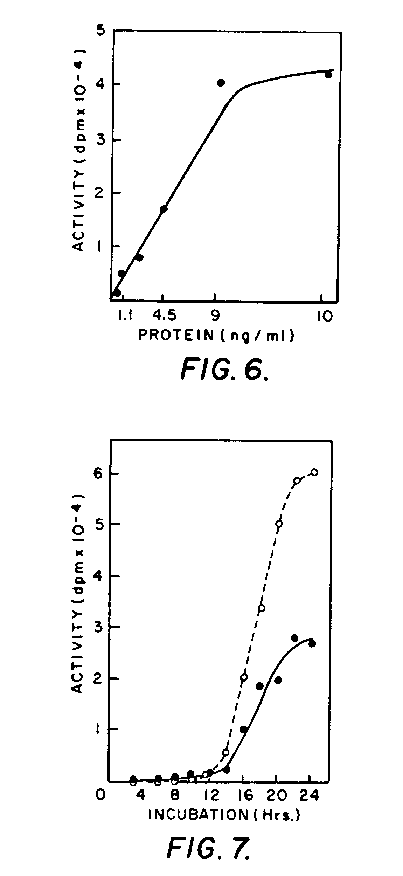

Increasing concentrations of purified PrGF were tested to determine the requirements of PrGF for biological activity. As shown in FIG. 6, mitogenic activity was detected at ~ 1 ng/ml PrGF. A linear dose-response was observed with maximum activity at ~ 16 ng/ml PrGF. Half-maximum activity was estimated at ~ 8 ng/ml.

## Time-Response Curve of PrGF

The time required for PrGF to initiate incorporation of [methyl-$^3$H]-thymidine into NRK-cell DNA was tested and compared with the mitogenic response of the cells to human serum. Five ng/ml of PrGF and 17% human serum were used; the results are shown in FIG. 7. DNA synthesis was stimulated by PrGF at ~ 14 hours after exposure of cells to PrGF and reached a maximum after ~ 22 hours. The time-response of mitogenic stimulation by PrGF was similar to that of human serum although the enhancement of [methyl-$^3$H]-thymidine incorporation by human serum was nearly double that observed with PrGF alone.

## Stability of PrGF to Heat, Reduction, and Proteases

The mitogenic activity of PrGF was completely retained after boiling purified PrGF (5 ng) for 5 min as shown in Table 3, below. Ten ng of purified PrGF was treated with 0.1 M $\beta$-mercaptoethanol for 3 min in boiling water. As shown in Table 3, PrGF lost essentially all mitogenic activity after treatment with $\beta$-mercaptoethanol. Two different proteases, trypsin and pronase, were tested with 5 $\mu$g of PrGF. Trypsin (100 $\mu$g/mld) reduced the mitogenic activity of PrGF to ~ 61% of original activity; pronase effectively destroyed all activity of PrGF. Neither inactivated trypsin (100 $\mu$g/ml) nor inactivated pronase (500 $\mu$g/ml) affected the biological activity of PrGF (Table 3).

In additional tests, PrGF was oxidized with sodium meta-periodate and reduced with [$^3$H]BH$_4$. Incorporation of [$^3$H] into purified PrGF was consistent with the labeling of carbohydrate, thereby further suggesting that PrGF is a glycoprotein. Purified PrGF was incubated also in cultures with preparations of osteoblasts from fetal rat calvaria. The purified growth factor stimulated the incorporation of [methyl-$^3$H]-thymidine into DNA of these isolated osteoblasts, providing initial evidence that PrGF may mediate the osteoblastic proliferation characteristic of metastatic prostatic cancer to bone.

EP 0 228 357 B1

Table 1
PURIFICATION OF PrGF FROM RAT PROSTATES

| Purification Step | Volume (ml) | Protein (mg) | Total Activity (dpm) | Yield (%) | (-fold) |
|---|---|---|---|---|---|
| Crude Extraction | 2700 | $6.99 \times 10^4$ | $2.32 \times 10^{10}$ | 100 | 1 |
| SP-Sephadex * | 6800 | $2.06 \times 10^{10}$ | $8.54 \times 10^9$ | 37 | 12.5 |
| DEAE-Sephadex * | 6150 | $3.08 \times 10^2$ | $8.89 \times 10^9$ | 38 | 87 |
| Ammonium Sulfate Precipitation | 50 | $2.55 \times 10^2$ | $6.45 \times 10^9$ | 28 | 93 |
| Sephadex G-75 * | 256 | $1.89 \times 10^1$ | $7.21 \times 10^8$ | 3.1 | 115 |
| DEAE-HPLC peak II | 24 | $2.74 \times 10^{-1}$ | $4.04 \times 10^8$ | 1.7 | 4442 |
| (peak I)[a] | (20) | (-) | $(1.58 \times 10^8)$ | (0.7) | (-) |
| (peak III)[b] | (16) | (-) | $(1.57 \times 10^8)$ | (0.7) | (-) |
| $C_{18}$-HPLC * | 10 | N.D.[c] | $3.16 \times 10^8$ | 1.4 | N.D. |
| TSK-2000 * | 14 | $4.48 \times 10^{-2}$ | $2.44 \times 10^8$ | 1.1 | 16385 |

All values were based on 900 g of rat prostatic tissue used as starting material. Recoveries were calculated by assuming that all of the initial mitogenic activity was PrGF.

[a] mitogenic activity released at lower salt concentration

[b] mitogenic activity released at higher salt concentration

[c] N.D. = not determined          [d] protein was quantitated by amino acid analysis

Table 2

AMINO ACID COMPOSITION[a] OF PrGF

| | Moles/25 kDa | Normalized moles/mole PrGF |
|---|---|---|
| Asx | 28.89 | 29 |
| Threonine | 10.32 | 10 |
| Serine | 13.38 | 13 |
| Glx | 23.62 | 24 |
| Proline | 27.27 | 27 |
| Glycine | 33.16 | 33 |
| Alanine | 17.98 | 18 |
| Cysteine | N.D.[b] | - |
| Valine | 15.18 | 15 |
| Methionine | 2.00 | 2 |
| Isoleucine | 7.98 | 8 |
| Leucine | 12.92 | 13 |
| Tyrosine | 11.03 | 11 |
| Phenylalanine | 6.08 | 6 |
| Histidine | 9.31 | 9 |
| Lysine | 6.69 | 7 |
| Arginine | 8.99 | 9 |
| Tryptophan | N.D.[b] | - |

[a] Amino acid analysis was performed using the methods of Ishido, Y., Fujita, T., and Asai, K. (1981) J. Chromatogr 204, 143-148.

[b] N.D. = not determined

Asx = Asparagine or Aspartic acid

Glx = Glutamine or Glutamic acid

Table 3

| TYPE OF TREATMENT | BIOLOGICAL ACTIVITY[a] |
|---|---|
| Control | 100 |
| Heat (100°C, 5 min) | 111 |
| Reduction (0.1M 2ME, 100°C, 3 min) | 11[b] |
| Proteases | |
| Trypsin (50 µg/ml) | 77 |
| Trypsin (100 µg/ml) | 61 |
| Inactivated Trypsin (100 µg/ml) | 94[c] |
| Pronase (100 µg/ml) | 1 |
| Pronase (500 µg/ml) | 1 |
| Inactivated Pronase (500 µg/ml) | 131[c] |

Effect of heat, $\beta$-mercaptoethanol, and proteases on PrGF activity. Five ng of purified PrGF was used for all experiments except 10 ng of PrGF was used for treatment with $\beta$-mercapto-ethanol (2ME). Details are described above.

[a] Results are expressed in percent of biological activity of control PrGF.

[b] Percent of biological activity of control PrGF, which was treated without $\beta$-mercaptoethanol but boiled and dialyzed the same way as the sample treated with $\beta$-mercaptoethanol.

[c] Inactivation of trypsin and pronase was performed by incubating enzymes in 0.1 M acetic acid in boiling water for 3 minutes prior to addition of PrGF.

The properties of PrGF have been compared with properties of various well-known growth factors mentioned hereinbefore. As shown in Table 4, below, PrGF appears to be unique and not related to these previously described growth factors. Growth factors are abbreviated as above, except NSILA = nonsuppressible insulin-like activity per Rinderknecht and Humbel, Proc. Natl. Acad. Sci. USA 73, 2365-2369 (1976).

**Table 4**
**Comparison of PrGF with Previously Described Growth Factors**

| Growth Factor | Origin | M.W. k dalton | Disulfide bonds | P.I. | Stability Heat | Acid |
|---|---|---|---|---|---|---|
| PDGF | Human Platelets | 28-31 | | 10.2 | stable | stable |
| EGF | Mouse Submaxillary Glands | 6 | + | 4.6 | stable | stable |
| FGF acidic | Bovine Pituitary Glands, Brain | 17 | - | 5.8 | labile | labile |
| FGF basic | | 15 | - | 8.5 | labile | labile |
| NGF | Mouse submandbular glands Guinea Pig, Rabbit Prostate Bovine seminal fluid | 13-15 | - | 8.5-1.0 | | |
| NSILA | Human Serum | 6 | - | 7.8-8.6 | stable | stable |
| ECGF | Bovine Brain | 75 | + | 4.6 | labile | labile |
| TGF α | Transformed Cell Lines | 5-7 | - | | stable | stable |
| TGF β | Transformed Cells, Human Platelets, Placenta Bovine Kidney | 25 | + | 5.0 | stable | stable |
| PrGF* | Rat Prostate Glands | 25 | + | 5.0 | stable | stable |

*Transforming Activity Absent

Various other examples will be apparent to the person skilled in the art after reading the disclosure herein withoug departing from the spirit and scope of the invention and it is intended that all such examples be included within the scope of the appended claims.

## Claims

1. A purified glycoprotein prostate growth factor having the following characteristics:

(a) molecular weight of about 25 kDa as determined by nonreduced sodium dodecylsulfate polyacrylamide gel electrophoresis;

(b) sensitivity to reduction by $\beta$-mercaptoethanol to form subunits of about 6-8 kDA with substantial loss of mitogenic activity;

(c) isoelectric point of about 5.0 as determined by isoelectric focusing;

(d) acid- and heat-stable;

(e) unstable to proteolytic action of trypsin and pronase;

(f) essentially free of transforming growth factor activity;

(g) amino acid composition about as follows:

| Amino Acid | moles/mole PrGF |
|---|---|
| Asx | 29 |
| Thr | 10 |
| Ser | 13 |
| Glx | 24 |
| Pro | 27 |
| Gly | 33 |
| Ala | 18 |
| Cys | - |
| Val | 15 |
| Met | 2 |
| Ile | 8 |
| Leu | 13 |
| Tyr | 11 |
| Phe | 6 |
| His | 9 |
| Lys | 7 |
| Arg | 9 |
| Trp | - |

wherein Cys and Trp are undetermined; and

(h) having mitogenic activity against normal rat kidney fibroblast (NRK) cells.

2. A process for preparing the prostate growth factor according to claim 1 having mitogenic activity against NRK cells comprising the steps of:

(a) homogenizing rat prostate cells;

(b) acid extracting the resulting homogenate;

(c) subjecting the acid extracted material to successive ion exchange column chromatography with SP-Sephadex C-25* and DEAE-Sephadex A-50*;

(d) concentrating the resulting active fractions by 70% ammonium sulfate precipitation;

(e) subjecting the concentrated fractions to gel permeation column chromatography with Sephadex G-75*;

(f) subjecting the resulting active fractions to ion exchange HPLC with a TSK-DEAE-5-PW* column;

(g) subjecting the resulting active fractions to reverse phase HPLC on a $C_{18}$* silica column;

(h) subjecting the resulting active fractions to HPLC on a TSK-G2000SW* gel permeation column; and

(i) recovering the resulting active prostate growth factor,

and wherein the symbol "*" means that the related term is a trade mark or a registered trade mark.

3. The method of stimulating the growth of fibroblast cells in vitro comprising subjecting said cells to a growth stimulating amount of a purified glycoprotein prostate growth factor having the following characteristics:

(a) molecular weight of about 25 kDa as determined by nonreduced sodium dodecylsulfate polyacrylamide gel electrophoresis;

(b) sensitivity to reduction by $\beta$-mercaptoethanol to form subunits of about 6-8 kDa with substantial loss of mitogenic activity;

(c) isoelectric point of about 5.0 as determined by isoelectric focusing;

(d) acid- and heat-stable;

(e) unstable to proteolytic action of trypsin and pronase;

(f) essentially free of transforming growth factor activity;

(g) amino acid composition about as follows:

| Amino Acid | moles/mole PrGF |
|------------|-----------------|
| Asx | 29 |
| Thr | 10 |
| Ser | 13 |
| Glx | 24 |
| Pro | 27 |
| Gly | 33 |
| Ala | 18 |
| Cys | - |
| Val | 15 |
| Met | 2 |
| Ile | 8 |
| Leu | 13 |
| Tyr | 11 |
| Phe | 6 |
| His | 9 |
| Lys | 7 |
| Arg | 9 |
| Trp | - |

wherein Cys and Trp are undetermined; and

(h) having mitogenic activity against NRK cells.

**Revendications**

1. Facteur de croissance prostatique glycoprotéinique purifié présentant les caractéristiques suivantes :

(a) une masse moléculaire d'environ 25 kDa, telle que déterminée par électrophorèse sur gel de dodécylsulfate de sodium-polyacrylamide non réduit;

(b) une sensibilité à la réduction par le $\beta$-mercaptoéthanol pour former des sous-unités d'environ 6-8 kDa avec une perte substantielle d'activité mitogène;

(c) un point isoélectrique d'environ 5,0 tel que déterminé par focalisation isoélectrique;

(d) stable aux acides et à la chaleur;

(e) instable à l'action protéolytique de la trypsine et de la pronase;

(f) pratiquement exempt d'activité de facteur de croissance transformant;

(g) une composition en amino-acides qui est approximativement la suivante:

14

| Amino-acide | Moles/mole de PrGF |
|---|---|
| Asx | 29 |
| Thr | 10 |
| Ser | 13 |
| Glx | 24 |
| Pro | 27 |
| Gly | 33 |
| Ala | 18 |
| Cys | - |
| Val | 15 |
| Met | 2 |
| Ile | 8 |
| Leu | 13 |
| Tyr | 11 |
| Phe | 6 |
| His | 9 |
| Lys | 7 |
| Arg | 9 |
| Trp | - |

dans laquelle Cys et Trp sont indéterminés; et

(h) possède une activité mitogène contre les fibroblastes de rein de rat normaux (NRK).

2. Procédé de préparation du facteur de croissance prostatique selon la revendication 1 ayant une activité mitogène contre les cellules NRK, comprenant les stades:

(a) homogénéiser des cellules prostatiques de rat;

(b) extraire par l'acide l'homogénéisat obtenu;

(c) soumettre la matière extraite par l'acide à des chromatographies sur colonne d'échange d'ions successives avec du SP-Sephadex C-25* et du DEAE-Sephadex A-50*;

(d) concentrer les fractions actives obtenues par précipitation par le sulfate d'ammonium à 70 %;

(e) soumettre les fractions concentrées à une chromatographie sur colonne à perméation de gel avec du Sephadex G-75*;

(f) soumettre les fractions actives obtenues à une HPLC d'échange d'ions avec une colonne de TSK-DEAE-5-PW*;

(g) soumettre les fractions actives obtenues à une HPLC en phase inversée sur une colonne de silice $C_{18}$*;

(h) soumettre les fractions actives obtenues à une HPLC sur une colonne à perméation de gel de TSK-G2000SW*; et

(i) récupérer le facteur de croissance prostatique actif obtenu,

le symbole "*" indiquant que le terme concerné est une marque ou une marque déposée.

3. Procédé de stimulation de la croissance de fibroblastes in vitro comprenant le fait de soumettre ces cellules à une quantité stimulant la croissance d'un facteur de croissance prostatique glycoprotéinique purifié présentant les caractéristiques suivantes :

(a) masse moléculaire d'environ 25 kDa, telle que déterminée par électrophorèse sur gel de dodécylsulfate de sodium-polyacrylamide non réduit;

(b) sensibilité à la réduction par le $\beta$-mercaptoéthanol pour former des sous-unités d'environ 6 à 8 kDa avec une perte substantielle d'activité mitogène;

(c) point isoélectrique d'environ 5,0 tel que déterminé par focalisation isoélectrique;

(d) stable aux acides et à la chaleur;

(e) instable à l'action protéolytique de la trypsine et de la pronase;

(f) pratiquement exempte d'activité de facteur de croissance transformant;

(g) une composition en amino-acides qui est approximativement la suivante :

15

| Amino-acide | Moles/mole de PrGF |
|---|---|
| Asx | 29 |
| Thr | 10 |
| Ser | 13 |
| Glx | 24 |
| Pro | 27 |
| Gly | 33 |
| Ala | 18 |
| Cys | - |
| Val | 15 |
| Met | 2 |
| Ile | 8 |
| Leu | 13 |
| Tyr | 11 |
| Phe | 6 |
| His | 9 |
| Lys | 7 |
| Arg | 9 |
| Trp | - |

dans laquelle Cys et Trp sont indéterminés; et

(h) elle présente une activité mitogène contre les cellules NRK.

**Patentansprüche**

1. Gereinigter Glykoprotein-Prostatawachstumsfaktor mit den folgenden Merkmalen:

(a) Molgewicht etwa 25 kDa, wie durch nicht-reduzierte Natriumdodecylsulfat-Polyacrylamidgelelektrophorese bestimmt;

(b) Empfindlichkeit auf Reduktion durch $\beta$-Mercaptoäthanol zum Bilden von Subeinheiten von etwa 6 bis 8 kDA mit wesentlichem Verlust von mitogener Aktivität,

(c) isoelektrischer Punkt etwa 5,0, bestimmt durch isoelektrisches Fokussieren,

(d) säure- und wärmestabil,

(e) instabil auf proteolytische Wirkung von Trypsin und Pronase,

(f) im wesentlichen frei von transformierender Wachstumsfaktoraktivität,

(g) Aminosäurezusammensetzung etwa wie folgt:

| Aminosäure | Mole/Mol PrGF |
|---|---|
| Asx | 29 |
| Thr | 10 |
| Ser | 13 |
| Glx | 24 |
| Pro | 27 |
| Gly | 33 |
| Ala | 18 |
| Cys | - |
| Val | 15 |
| Met | 2 |
| Ile | 8 |
| Leu | 13 |
| Tyr | 11 |
| Phe | 6 |
| His | 9 |
| Lys | 7 |
| Arg | 9 |
| Trp | - |

wobei Cys und Trp nicht bestimmt sind, und

(h) mit mitogener Aktivität gegen normale Rattennierenfibroblasten (NRK)-Zellen.

2. Verfahren zum Herstellen des Prostatawachstumsfaktors nach Anspruch 1 mit mitogener Aktivität gegen NRK-Zellen umfassend die folgenden Schritte:

(a) Homogenisieren von Rattenprostatazellen,

(b) Säureextraktion des erhaltenen Homogenats,

(c) Unterwerfen des säureextrahierten Materials aufeinanderfolgender Ionenaustauschsäulenchromatographie mit SP-Sephadex C-25* und DEAE-Sephadex A-50*,

(d) Konzentrieren der erhaltenen aktiven Fraktionen durch Ausfällung mit 70 %igem Ammoniumsulfat,

(e) Unterwerfen der konzentrierten Fraktionen Gelpermeationssäulenchromatographie mit Sephadex G-75*,

(f) Unterwerfen der erhaltenen aktiven Fraktionen Ionenaustausch-HPLC mit einer TSK-DEAE-5-PW*-Säule,

(g) Unterwerfen der erhaltenen aktiven Fraktionen Umkehrphasen-HPLC auf einer $C_{18}$*-Kieselerdesäule,

(h) Unterwerfen der erhaltenen aktiven Fraktionen HPLC auf einer TSK-G2000SW*-Gelpermeationssäule und

(i) Gewinnen des erhaltenen aktiven Prostatawachstumsfaktors,

wobei das Symbol "*" bedeutet, daß der betreffende Ausdruck ein Handelsname oder eine registrierte Marke ist.

3. Verfahren zum Stimulieren des Wachstums von Fibroblastenzellen in vitro, das das Aussetzen dieser Zellen an eine wachstumsstimulierende Menge eines gereinigten Glykoprotein-Prostatawachstumsfaktors mit folgenden Merkmalen

(a) Molgewicht etwa 25 kDa, wie durch nicht-reduzierte Natriumdodecylsulfat-Polyacrylamidgelelektrophorese bestimmt;

(b) Empfindlichkeit auf Reduktion durch $\beta$-Mercaptoäthanol zum Bilden von Subeinheiten von etwa 6 bis 8 kDA mit wesentlichem Verlust von mitogener Aktivität,

(c) isoelektrischer Punkt etwa 5,0, bestimmt durch isoelektrisches Fokussieren,

(d) säure- und wärmestabil,

(e) instabil auf proteolytische Wirkung von Trypsin und Pronase,

(f) im wesentlichen frei von transformierender Wachstumsfaktoraktivität,

(g) Aminosäurezusammensetzung etwa wie folgt:

| Aminosäure | Mole/Mol PrGF |
|:----------:|:-------------:|
| Asx | 29 |
| Thr | 10 |
| Ser | 13 |
| Glx | 24 |
| Pro | 27 |
| Gly | 33 |
| Ala | 18 |
| Cys | - |
| Val | 15 |
| Met | 2 |
| Ile | 8 |
| Leu | 13 |
| Tyr | 11 |
| Phe | 6 |
| His | 9 |
| Lys | 7 |
| Arg | 9 |
| Trp | - |

wobei Cys und Trp nicht bestimmt sind, und
(h) mit mitogener Aktivität gegen NRK-Zellen umfaßt.

FIG. I.

FIG. 2.

FIG. 3.

FIG. 4.

FIG. 5.

FIG. 6.

FIG. 7.